# EUROPÄISCHE PATENTANMELDUNG

(11) **EP 0 864 331 A2**
(43) Veröffentlichungstag der Anmeldung: **16.09.1998**
(21) Anmeldenummer: 98103550.4
(22) Anmeldetag: 28.02.1998
(51) Int. Cl.: A61L 15/50

(54) **Darreichung zur Reduzierung der Klebkraft von Klebebändern**

(30) Priorität: 14.03.1997 DE 19710543
(71) Anmelder: Beiersdorf Aktiengesellschaft, 20245 Hamburg (DE)
(72) Erfinder: Himmelsbach, Peter, 21614 Buxtehude (DE); Pietsch, Hanns, Dr., 20148 Hamburg (DE); Knieler, Roland, Dr., 22529 Hamburg (DE)

(57) **Zusammenfassung**

Darreichung zur Reduzierung der Klebkraft von Klebebändern, bestehend aus mindestens einem Penetranten und mindestens einem klebkraftreduzierenden Wirkstoff, welcher hautverträglich ist und aktiv zur Hautpflege betragen kann, wobei der klebkraftreduzierende Wirkstoff mindestens eine Kohlenwasserstoffkette der Form (CₙHₘ) aufweist, wobei n die Anzahl der Kohlenstoffatome in der Kette ist und einen Wert von größer als 6 aufweist, und wobei der klebstoffreduzierende Wirkstoff einen primären Irritationsindex von weniger als 3, besonders bevorzugt 0, aufweist.

## Beschreibung

Die Erfindung betrifft eine Darreichung zum zerstörungs- und rückstandsfreien Ablösen von Klebebändern, insbesondere medizinischen Pflasterbinden.

Starkklebende Pflasterbinden, insbesondere orthopädische Bandagen, lassen sich in der Regel nur schwer nach der Anwendung ablösen. Fallweise kommt es beim Ablösen zu mechanischen Verletzungen der Haut oder zur Epilation. Um ein schmerzfreies Ablösen zu ermöglichen, werden sogenannte Tape-Remover eingesetzt. Diese erleichtern das Ablösen, wirken jedoch nur bedingt für spezielle Klebemassensysteme.

Nachteilig kann sich von den bekannten Systemen das regelmäßige Anwenden auf Haut auswirken, da dieses zum Austrocknen der Haut führt. Bislang bekannte Tape-Remover wirken kaum oder gar nicht hautpflegend.

Generell sind neben der Funktionalität, also der Wirksamkeit des Systems, auch ein schnelles Einwirken und guten Dosieren wünschenswert. Insbesondere für orthopädische Bandagen ist es notwendig, daß der Tape-Remover auch durch mehrere Schichten von Klebestreifen wirkt.

Da insbesondere orthopädische Bandagen, wie zum Beispiel bei funktionellen Tape-Verbänden, der Verband zum Verbandswechel entfernt wird, ist es auch zwingend notwendig, daß die wirkende Substanz unmittelbar nach der Abnahme des Verbandes ein erneutes Verkleben mit einem neuen Streifen nicht behindert.

Es sind Tape-Remover für Klebemassensysteme auf Basis von Zn-Kautschuk-Klebemassen auf dem Markt gängig. Nachteilig dieser Tape-Remover ist, daß sie nicht aktiv hautpflegend sind und nicht für alle Klebemassensysteme, insbesondere für spezielle Heißschmelzklebemassen auf Basis von Blockcopolymeren, tauglich sind.

JO173437 A beschreibt eine Verbindung auf Basis eines R-COOR1 Fettsäureesters, welcher in organischen Lösemitteln verteilt wird, wobei mindestens eine Gruppe eine lange alkyl. Kohlenwasserstoffkette aufweist. Sei diesem System ist schon durch die Auswahl der Wirksubstanz die Funktionalität für einen universellen Einsatz auf verschiedene Klebesysteme äußerst eingeschränkt.

DE P 16 69 304.2 beschreibt ein Verfahren zur rückstandsfreien Ablösung von Pflastern und anderen Klebeprodukten. Die aufgeführten Beispiele enthalten aber keine hautpflegende Substanz, teilweise sind diese sogar heute als gesundheitsschädigend einzustufen. Die bevorzugten wirkenden Bestandteile sind Amide und Sulfoxide. Eine hautpflegende Eigenschaft wurde nicht beschrieben, ebenfalls nicht die universelle Wirkung auf verschiedene Klebesysteme.

US 4 867 981 beschreibt ebenfalls eine Klebeband ablösende Zusammensetzung, welche aus verschiedenen hydrophilen und hydrophoben Bestandteilen sich zusammen setzt. Eine hautpflegende Eigenschaft wurde nicht beschrieben, ebenfalls nicht die universelle Wirkung auf verschiedene Klebesysteme.

Aufgabe der Erfindung war es, eine Darreichung zur Reduzierung der Klebkraft zur Verfügung zu stellen, die die aus dem Stand der Technik bekannten Nachteile nicht aufweist und die insbesondere hautpflegend wirkt.

Gelöst wird diese Aufgabe durch eine Darreichung, wie sie im Anspruch 1 beschrieben ist. Gegenstand der Unteransprüche sind dabei vorteilhafte Weiterbildungen.

Demgemäß besteht die Darreichung zur Reduzierung der Klebkraft von Klebebändern aus mindestens einem Penetranten und mindestens einem klebkraftreduzierenden Wirkstoff, welcher hautverträglich ist und aktiv zur Hautpflege beitragen kann, wobei der klebkraftreduzierende Wirkstoff mindestens eine Kohlenwasserstoffkette der Form (CₙHₘ) aufweist, wobei n die Anzahl der Kohlenstoffatome in der Kette ist und einen Wert von größer als 6 aufweist, und wobei der klebstoffreduzierende Wirkstoff einen primären Irritationsindex von weniger als 3, besonders bevorzugt 0, aufweist.

In einer bevorzugten Ausführungsform der Erfindung weist der Wirkstoff einen H₅₀-Wert von größer 1000 mg/L, bevorzugt größer 3000 mg/L, besonders bevorzugt größer 10000 mg/L auf.

Vorzugsweise liegt der Wirkstoff zu einem Anteil von kleiner 99 Gew.-%, bevorzugt kleiner 50 Gew.-%, besonders bevorzugt 0,01 Gew.-% bis 10 Gew.-% in der Darreichung vor.

Der Wirkstoff ist weiter vorzugsweise ein Kohlenwasserstoff, bevorzugt ein Kohlenwasserstofföl oder Paraffin, ein Ether, ein Alkohol, insbesondere Tetradecanol oder Hexadodecanol, ein Ester mit mindestens einer aromatischen Gruppe oder ein Triglycerid.

Vorzugsweise reduziert die erfindungsgemäße Darreichung die Klebkraft eines Klebestreifens auf Stahl beziehungsweise auf Haut um mindestens 30%. Damit wird ein zerstörungsfreies beziehungsweise schmerzfreies und epilationsfreies Ablösen des Klebestreifens ermöglicht.

Weiter sind die Bestandteile der erfindungsgemäßen Darreichung so ausgewählt, daß sie auch bei einer mehrlagigen Anwendung bis auf den Untergrund durchdringen können. Dies wird vorzugsweise durch eine gezielte Viskositätseinstellung der Darreichung und/oder des Wirkstoffs erreicht.
Hierbei können sowohl eine newton'sch fließende Darreichung, als auch eine nicht newton'sch fließende Darreichung je nach Anwendungsfall vorteilhaft sein. Beispielhaft sind hier das pseudoplastische oder plastische, das tixotrope und rheopexe Fließverhalten für solche Darreichungen genannt. Gleiches trifft auf den Wirkstoff zu.

Vorteilhaft ist es, wenn sich für die Anwendung der Darreichung eine Viskosität η bei einer Temperatur von 25 °C und einem Schergefälle von 1*1/s von weniger als 10 Pas, bevorzugt von weniger als 1 Pas, ergibt.

Für das Dosieren der Darreichung hat sich zudem noch gezeigt, daß sich Darreichungen mit einem plastischen Fließverhalten einfacher und ohne Verluste applizieren lassen, als Darreichungen ohne plastisches Fließverhalten. Die Fließgrenze τₖᵣᵢₜ und die kritische Viskosität ηₖᵣᵢₜ werden vorteilhafterweise so gewählt, daß sie auch bei einer mehrlagigen Anwendung bis auf den Untergrund durchdringen können. Vorteilhaft hat sich hier ergeben, daß die kritische ηₖᵣᵢₜ bei einer Temperatur von 25 °C immer geringer ist als 5,0*10⁴ Pas und die Schubspannung τₖᵣᵢₜ bei ηₖᵣᵢₜ und 25 °C immer kleiner 25 Pa. Besonders bevorzugt sind Darreichungen, bei denen bei einer Temperatur von 25 °C die kritische Viskosität ηₖᵣᵢₜ immer geringer ist als 2*10⁴ Pas und die Schubspannung τₖᵣᵢₜ bei ηₖᵣᵢₜ und 25°C immer geringer als 15 Pa ist.
Durch ein leichtes Einreiben einer solchen Darreichung wird die Viskosität dann so abgesenkt, daß ein Penetrieren durch die Klebebandschichten möglich ist.

Für die Abprüfung der Hautverträglichkeit sind etliche Methoden wissenschaftlich beschrieben. Dieses gilt auch für die aktive Hautpflege von Substanzen oder Mischungen. Nachfolgend sollen nur zwei Methoden, der HET-Test und der RBC-Test beispielhaft beschrieben werden.

Bei dem HET-Test (Horst Spielmann et al, Results of Validation Study in Germany on Two *In Vitro* Alternatives to the Draize Eye Irritation, the HET-CAM Test and the 3T3 NRU Cytotoxity Test, ALTA 24, 741-858, 1996) werden vorbehandelte Hühnereier mit den entsprechenden Wirksubstanzen bzw. Darreichungen auf spezielle Art und Weise zusammengefügt.
Nach einer festgelegten Zeit nach der Applikation werden die Eintrittszeitpunkte von drei Reaktionstypen bestimmt. Diese führen über eine empirische Formel zu den primären Irritationsindizes, einer Klassifizierung der Wirksubstanzen oder Darreichungen. Stark redende Substanzen haben einen hohen primären Irritationsindex. Eine nur schwach oder nicht reizende Substanz oder Darreichung besitzen einen primären Irritationsindex von kleiner als drei.

Durch den RBC-Test nach Pape et. al. und Balls et. al. (Pape W.J. W. et al; Validation of the red blood cell test system as in vitro assay for the rapid screening of irritation potentials of surfactants, Mol Toxical. 1987; 1, 525-536; Balls M. et al, The EC/HO International Validation Study on Alternatives to the Eye Irritation Test; Toxicology in vitro, 1995, 9, 871-929) lassen sich ebenfalls Reizpotentiale bestimmen. In einer Reihe mit steigenden Konzentration werden hierbei die zu untersuchende Wirksubstanzen oder Darreichungen wird mit einem definierten Aliquot isolierter Kalbserythrozyten inkubiert und analysiert. Der daraus resultierende Hämolysegrad ermöglicht die Berechnung des H₅₀-Wert (mg/L), also die Konzentration bei der 50% der Hämoglobins freigesetzt wurden.
Ist dieser H₅₀-Wert größer als 1000 mg/L, bevorzugt größer 3000 mg/L, besonders bevorzugt größer 10000 mg/L wird das Reizpotential als gering eingestuft.

Der Anteil aller Substanzen, die ein Reizpotential haben, sollte in der Darreichung immer geringer sein als 300 mg/kg, bevorzugt 200 mg/kg.

Im folgenden soll die erfindungsgemäße Darreichung mittels mehrerer Beispiele näher erläutert werden, ohne daß damit der Erfindungsgegenstand unnötig eingeschränkt werden soll.

### Beispiel 1

### Zusammensetzung:

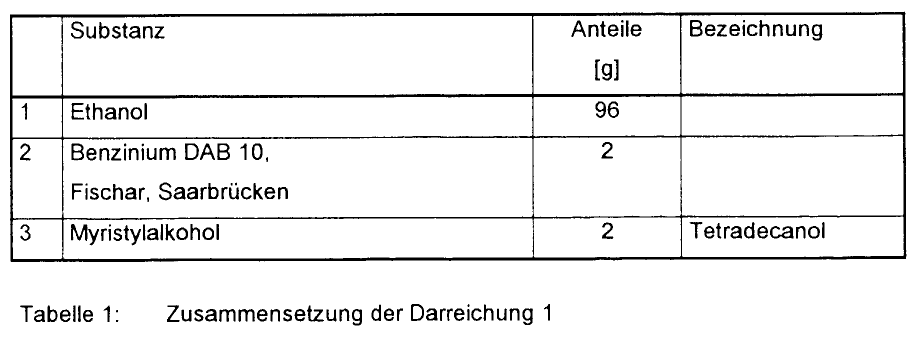

Die Herstellung der Darreichung 1 erfolgte in einem Becherglas. Dazu wurde das Tetradecanol in das Benzinium gegeben, anschließend das Ethanol unter ständigem Rühren in die Mischung zugetropft.

Die so hergestellte Darreichung hatte eine Viskosität η von η = 2,95*10⁻³ Pas bei einer Temperatur von 25 °C und einem Schergefälle von 100*1/s. Sie wies somit ein quasi newton'sches Fließverhalten auf.

Das Tetradecanol wies einen H₅₀-Wert von ca. 1900 mg/L auf.

Um die mittels der Darreichung 1 erzielbare Reduzierung der Klebkraft von Klebebändern festzustellen, wurden selbstklebend beschichtete Trägermaterialien untersucht. Als Klebmassen wurden dazu Acrylatklebmassen, Klebmassen auf Zn-Kautschukbasis und auf Basis von SEBS-Blockcopolymeren verwendet.

Zunächst wurden die selbstklebend beschichteten Trägermaterialien in Abschnitten mit einer Fläche von 2 cm x 5 cm auf Stahl verklebt. Anschließend wurden die Abschnitte mit der erfindungsgemäßen Darreichung, hier also die Darreichung 1, bestrichen. Nach einer Einwirkungsdauer von 10 Sekunden wurden die Klebkräfte der Abschnitte auf Stahl gemessen, und zwar nach der 180°-Methode. Zuvor wurden die Klebkräfte gleicher Abschnitte auf Stahl nach der 180°-Methode bestimmt, ohne daß eine Darreichung eingesetzt wurde.

In der anschließenden Tabelle 2 sind die gemessenen Werte dargestellt.

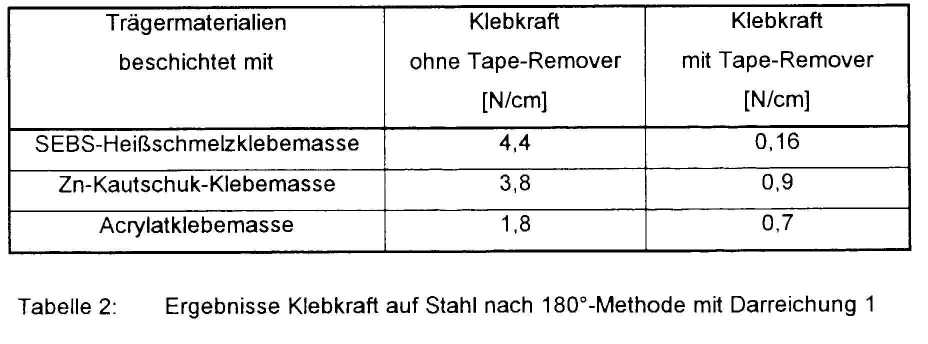

Demgemäß reduzierte die Darreichung 1 die Klebkraft auf Stahl um mindestens 30%, wobei die größte Reduzierung bei Trägermaterialien zu erzielen war, die mit einer SEBS-Heißschmelzklebemasse beschichtet waren.

Gleiches traf auch für Trägermaterialabschnitte zu, die auf der Haut von Probanden verklebt waren.

Weiterhin zeigte die Haut auch nach zehn Anwendungen keine Irritationen. Die Klebmasse wurde dabei nicht angelöst, es kam auch nicht zu einem Transfer der Klebemasse auf die Haut. Bei allen selbstklebend beschichteten Trägermaterialien konnte ein erneutes Verkleben der Abschnitte auf die selben Hautbereiche erfolgen.

Das subjektive Empfinden von Probanden bescheinigte der Darreichung 1 eine kühlende Pflegewirkung.

### Beispiel 2

### Zusammensetzung:

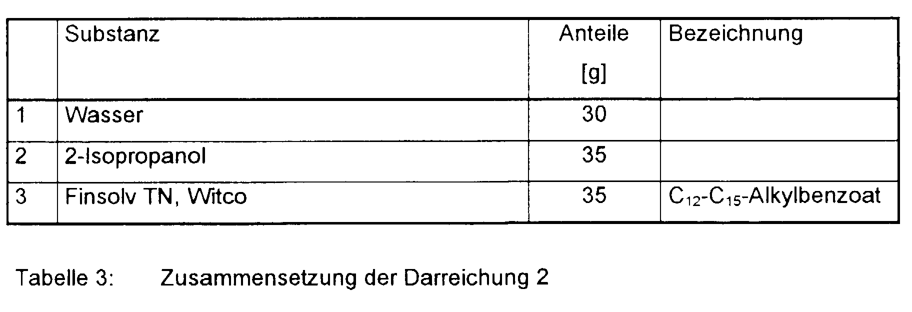

Die Herstellung der Darreichung 2 erfolgte in einem Becherglas. Dazu wurde das Finsolv TN in das 2-Isopropanol gegeben, anschließend das Wasser unter ständigem Rühren in die Mischung zugetropft.

Das C₁₂-C₁₅-Alkylbenzoat weist einen Rezindex von 0 auf.

Entsprechend Beispiel 1 wurde auch in Beispiel 2 eine gleiche Versuchsreihe mit der Darreichung 2 und unterschiedlich beschichteter Trägermaterialien durchgeführt.

Es ergaben sich folgende Ergebnisse:

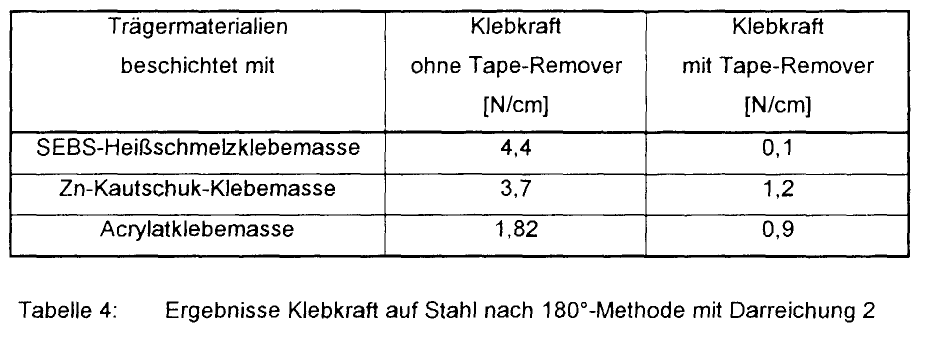

Demgemäß reduzierte die Darreichung 2 die Klebkraft auf Stahl sogar bis zu 95%, wobei wiederum die größte Reduzierung bei den Trägermaterialien mit einer SEBS-Heißschmelzklebemasse zu erzielen war.

Gleiches traf auch für Trägermaterialabschnitte zu, die auf der Haut von Probanden verklebtwaren.

Weiterhin zeigte die Haut auch nach zehn Anwendungen keine Irritationen. Die Klebmasse wurde dabei nicht angelöst, es kam auch nicht zu einem Transfer der Klebemasse auf die Haut. Bei allen selbstklebend beschichteten Trägermaterialien konnte ein erneutes Verkleben der Abschnitte auf die selben Hautbereiche erfolgen.

Das subjektive Empfinden von Probanden bescheinigte der Darreichung 2 eine kühlende, glättende Pflegewirkung.

### Beispiel 3

### Zusammensetzung:

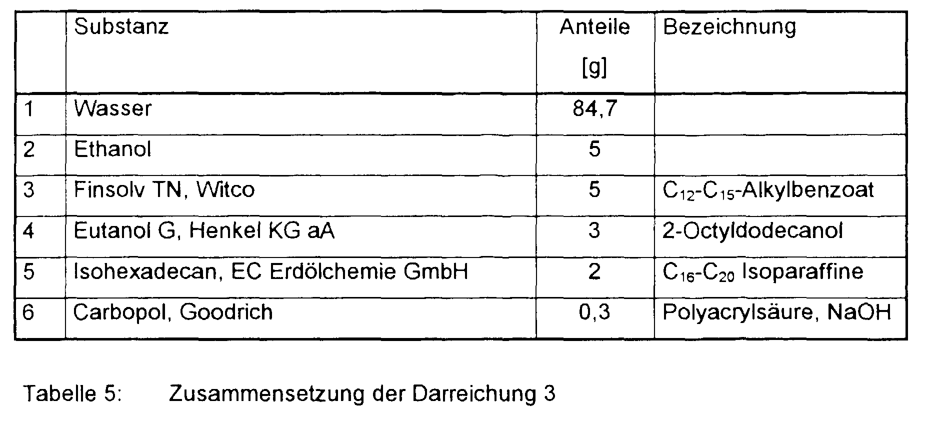

Die Herstellung der Darreichung 3 erfolgte, indem Isohexadecan, Finsolv TN, Eutanol G und Carbopol in einem offenen Rührgefäß vermengt wurden. Die parallel hergestellte Mischung aus Ethanol und Wasser wurde bei 80 °C tropfenweise in das Rührgefäß zudosiert.

Anschließend wurden die Substanzen in einen Homogenisator verrührt, bis eine homogene Mischung hergestellt war.

Die Wirksubstanzen wurden in einer Mischung eingesetzt. Isohexadecan, Finsolv TN, Eutanol G hatten einen Reizindex von 0.

Die Darreichung 3 wies plastisches Fließverhalten auf, die kritische Fließviskosität betrug ηₖᵣᵢₜ = 1,04*10⁴ Pas, die kritische Schubspannung τₖᵣᵢₜ = 10 Pa.

Darreichung 3 durchdrang die gemäß Beispiel 1 hergestellte, selbstklebend beschichtete Trägermaterialien in weniger als 30 Sekunden.

Es ergaben sich die folgenden Ergebnisse:

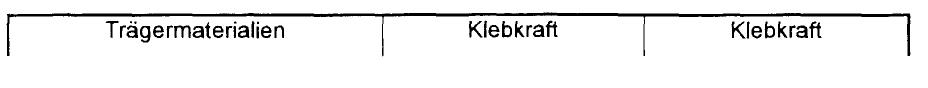

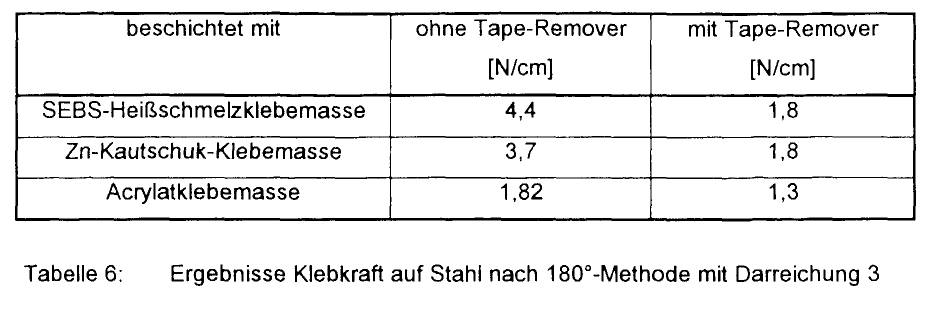

Demgemäß reduzierte die Darreichung 3 die Klebkraft auf Stahl um bis zu 65%, wobei erneut die größte Reduzierung bei SEBS-Heißschmeizklebemasse festzustellen war.

Gleiches traf auch für Trägermaterialabschnitte zu, die auf der Haut von Probanden verklebtwaren.

Werterhin zeigte die Haut auch nach zehn Anwendungen keine Irritationen. Die Klebmasse wurde dabei nicht angelöst, es kam auch nicht zu einem Transfer der Klebemasse auf die Haut. Bei allen selbstklebend beschichteten Trägermaterialien konnte ein erneutes Verkleben der Abschnitte auf die selben Hautbereiche erfolgen.

Das subjektive Empfinden von Probanden bescheinigte der Darreichung 3 eine kühlende, glättende Pflegewirkung.

## Patentansprüche

1. Darreichung zur Reduzierung der Klebkraft von Klebebändern, bestehend aus mindestens einem Penetranten und mindestens einem klebkraftreduzierenden Wirkstoff, welcher hautverträglich ist und aktiv zur Hautpflege betragen kann, wobei der klebkraftreduzierende Wirkstoff mindestens eine Kohlenwasserstoffkette der Form (CₙHₘ) aufweist, wobei n die Anzahl der Kohlenstoffatome in der Kette ist und einen Wert von größer als 6 aufweist, und wobei der klebstoffreduzierende Wirkstoff einen primären Irritationsindex von weniger als 3, besonders bevorzugt 0, aufweist.

2. Darreichung nach Anspruch 1, dadurch gekennzeichnet, daß der Wirkstoff einen H₅₀-Wert von größer 1000 mg/L, bevorzugt größer 3000 mg/L, besonders bevorzugt größer 10000 mg/L aufweist.

3. Darreichung nach den Ansprüchen 1 oder 2, dadurch gekennzeichnet, daß der Wirkstoff zu einem Anteil von kleiner 99 Gew.-%, bevorzugt kleiner 50 Gew.-%, besonders bevorzugt 0,01 Gew.-% bis 10 Gew.-% in der Darreichung vorliegt.

4. Darreichung nach den Ansprüchen 1 bis 3, dadurch gekennzeichnet, daß der Wirkstoff ein Kohlenwasserstoff, bevorzugt ein Kohlenwasserstofföl oder Paraffin, ein Ether, ein Alkohol, insbesondere Tetradecanol oder Hexadodecanol, ein Ester mit mindestens einer aromatischen Gruppe oder ein Triglycerid ist.

5. Darreichung nach den Ansprüchen 1 bis 4, dadurch gekennzeichnet, daß die Klebkraft von Klebebändern auf Stahl und auf Haut um mindestens 30% reduziert wird.

6. Darreichung nach den Ansprüchen 1 bis 5, dadurch gekennzeichnet, daß der Wirkstoff und/oder die Darreichung ein newton'sches, ein pseudoplastisches, strukturviskoses oder ein tixotrophes Fließverhalten aufweisen.

7. Darreichung nach den Ansprüchen 1 bis 6, dadurch gekennzeichnet, daß die Darreichung eine Viskosität η von weniger als 10 Pas bei einer Temperatur von 25 °C und einem Schergefälle von 1*1/s aufweist.

8. Darreichung nach den Ansprüchen 1 bis 7, dadurch gekennzeichnet, daß die Darreichung eine kritische Viskosität ηₖᵣᵢₜ von weniger als 5,0*10⁴ Pas, bevorzugt weniger als 2,0*10⁴ Pas, bei einer Temperatur von 25 °C und eine Schubspannung τₖᵣᵢₜ von weniger als 25 Pa, bevorzugt weniger als 15 Pa, bei ηₖᵣᵢₜ und 25 °C aufweist.
